# EUROPEAN PATENT SPECIFICATION

(11) **EP 2 675 490 B1**
(45) Date of publication and mention of the grant of the patent: **03.10.2018**
(21) Application number: 12707419.3
(22) Date of filing: 14.02.2012
(51) Int. Cl.: A61L 27/46, A61L 27/56

(54) **NON-RESORBABLE POLYMER - CERAMIC COMPOSITE IMPLANT MATERIALS**
IMPLANTATMATERIALIEN AUS NICHT-RESORBIERBAREM POLYMERKERAMIKVERBUNDSTOFF
MATÉRIAUX D'IMPLANTS COMPOSITES POLYMÈRE-CÉRAMIQUE, NON RÉSORBABLES

(30) Priority: 14.02.2011 US 201161442656 P; 06.02.2012 US 201261595418 P
(43) Date of publication of application: 25.12.2013
(73) Proprietor: Biomet Manufacturing, LLC, Warsaw, IN 46582 (US)
(72) Inventor: PONTICIELLO, Michael, Mission Viejo, CA 92691 (US); COALE, Bradford, J., Vernon, NJ 07462 (US); D'ANTONIO, Paul, Morristown, NJ 07960 (US); HERNANDEZ, Joseph, M., Torrence, CA 90505 (US)
(74) Representative: Manitz Finsterwald Patentanwälte PartmbB
(86) International application number: PCT/US2012/024984
(87) International publication number: WO 2012/112499

(56) References cited:
- EP-A1- 1 604 696
- WO-A2-02/053105
- US-A1- 2011 022 180

## Description

### BACKGROUND

The present technology relates to materials useful in orthopedic surgery, including orthopedic implants.

The human musculoskeletal system is composed of a variety of tissues including bone, ligaments, cartilage, muscle, and tendons. Tissue damage or deformity stemming from trauma, pathological degeneration, or congenital conditions often necessitates surgical intervention to restore function. During these procedures, surgeons can use orthopedic implants to restore function to the site and facilitate the natural healing process. Depending on the site of implantation and the desired treatment, such implants may be load-bearing (i.e., capable of supporting surrounding structures without significant deformity under typical physiological conditions). It may also be desirable for such implants to be integrated into existing natural tissues, such as by ingrowth of natural bone into the implant material.

A variety of polymer and ceramic materials have also been used as an implant material. For example, such materials have been used in fracture fixation, bone grafting, spinal fusion, soft tissue repair, and deformity correction. Specific structures include implants such as screws, plates, pins, rods, and intervertebral spacers. The specific composition of these materials can affect the physiological properties of the implants. For many applications it may be desired for such implants to be both load- bearing as well as capable of integration with surrounding natural tissue. However, many such materials do not offer such a combination of properties, for example having osteoconductive and/or osteoinductive properties, but lacking load-bearing capacity.

US 2011/0022180 A1 discloses an implantable medical device including a body having a porous matrix with a series of interconnected macropores defined by a plurality of interconnected struts each including a hollow interior. At least a portion of the macropores is filled with a filler material and an external surface of the body includes a plurality of openings communicating with hollow interior of at least a portion of the plurality of interconnected struts.

EP 1 604 696 A1 discloses a composite scaffold for the ingrowths of tissue. The scaffold comprises a ceramic phase having a first plurality of pores and a polymer phase having a second plurality of the pores. The polymer phase is attached to the ceramic phase and is infused at least partially into the plurality of pores in the ceramic phase.

In WO 02/053105 A2 a biomedical implant for bone substitution and replacement applications is disclosed. The biocompatible implant comprises a matrix comprising a resorbable thermoplastic ceramic composition.

### SUMMARY

The present technology provides materials, compositions, devices and methods relating to polymer constructs and composites that comprise a non-resorbable polymer, such as polyetheretherketone (PEEK). The constructs and composites comprise interconnected struts, which may define a coralline structure.

In various embodiments, the present technology provides orthopedic implant composites comprising: a first phase comprising a ceramic that is coral or that is derived from coral via a hydrothermal conversion of calcium carbonate with calcium phosphate, wherein the first phase has a porous coralline structure; and a second phase comprising a non-resorbable polymer; wherein each of the first and second phases have an interconnected strut structure and are substantially continuous through the composite, wherein the orthopedic composite has a compressive strength of 50 MPa to 150 MPa. The ceramic may be calcium phosphate, calcium carbonate, or mixtures thereof. The composites may also contain a bioactive material, such as peptides, cytokines, and antimicrobials. In some embodiments, the implant comprises a core containing the composite, and a porous layer containing non-resorbable polymer that is contiguous with the core. The implant may also comprise a non-porous component containing the non- resorbable polymer that is contiguous with a surface of the core, a surface of the porous layer (if present), or both.

The present technology also provides methods of making bone graft composites, comprising infusing a porous ceramic body, having a plurality of interconnected channels, with a non-resorbable polymer. The resulting composite may comprise a first phase of the ceramic and a second phase of the non-resorbable polymer, wherein the first and second phases are substantially continuous through the composite. The infusing may involve placing the ceramic body into a mold and injecting the non- resorbable polymer into the mold so as to fill one or more of the channels. In some embodiments, the ceramic body defines a void in the mold, so that the composite comprises two components, the first component comprising the ceramic body having one or more channels filled with the polymer, and the component comprising non-porous polymer.

### BRIEF DESCRIPTION OF THE DRAWINGS

Figure 1 is a perspective view of a porous structure of the present technology.
Figure 2 is a perspective view of a composite of the present technology.
Figure 3a is a microphotograph of a cross-section of a composite of the present technology. Figure 3b is a scanning electron micrograph of a composite of the present technology.
Figure 4 is a photograph of a spinal spacer implant of the present technology.
Figure 5 is a perspective view of a spinal spacer implant of the present technology.
Figure 6 is a perspective view of a spinal spacer implant of the present technology, comprising a composite of the present invention and a solid non-porous component.
Figure 7 is a perspective view of a spinal spacer implant of the present technology, comprising a composite of the present invention and a solid non-porous component.
Figure 8 is a perspective view of a spinal spacer implant of the present technology, comprising a composite of the present invention and a solid non-porous component.
Figure 9 is a flow chart exemplifying methods of the present technology.
Figure 10 is a photograph of a cross-section of a molded implant material of the present technology.

It should be noted that the figures set forth herein are intended to exemplify the general characteristics of materials, compositions, devices, and methods among those of the present technology, for the purpose of the description of certain embodiments. These figures may not precisely reflect the characteristics of any given embodiment, and are not necessarily intended to fully define or limit specific embodiments within the scope of this technology.

### DESCRIPTION

The following description of technology is merely exemplary in nature of the composition, manufacture and use of one or more inventions, and is not intended to limit the scope, application, or uses of any specific invention claimed in this application or in such other applications as may be filed claiming priority to this application, or patents issuing therefrom. A non-limiting discussion of terms and phrases intended to aid understanding of the present technology is provided at the end of this Detailed Description.

The implant constructs of the present technology comprise a non-resorbable polymer and, in various composite embodiments, a ceramic. (It should be noted that, in general, embodiments of the present technology comprising a single-phase material are referred to as "constructs" whereas embodiments comprising a multi-phase material are referred to as "composites." However, the terms "construct" and "composite" may be used interchangeably in many contexts of this disclosure, and are not intended to limit the specific composition or architecture of any described embodiment.) As further discussed below, the implant constructs, composites, and devices may be used for the treatment of bony or other tissue defects in humans or other animal subjects. Accordingly, specific materials to be used in composites and constructs of the present technology must be biomedically acceptable. Such a "biomedically acceptable" material is one that is suitable for use with humans and/or animals without undue adverse side effects (such as toxicity, irritation, and allergic response) commensurate with a reasonable benefit/risk ratio.

### Materials and Composites

Non-resorbable polymers among those useful herein include polymers that do not substantially resorb, dissolve or otherwise degrade after implantation in a human or animal subject, under typical physiological conditions. Such polymers include polyaryl ether ketone (PAEK) polymers (such as polyetherketoneketone (PEKK), polyetheretherketone (PEEK), and polyetherketoneetherketoneketone (PEKEKK)), polyolefins (such as ultra-high molecular weight polyethylene, which may be crosslinked, and fluorinated polyolefins such as polytetrafluorethylene (PTFE)), polyesters, polyimides, polyamides, polyacrylates (such as polymethylmethacrylate (PMMA)), polyketones, polyetherimide, polysulfone, polyurethanes, and polyphenolsulfones. In various embodiments, a preferred polymer comprises, or consists of, polyetheretherketone (PEEK). A commercially available PEEK is sold as PEEK-OPTIMA® LT3 by Invibio, Inc. (West Conshohocken, Pennsylvania, USA).

Fillers can be added to a polymer, copolymer, polymer blend, or polymer composite to reinforce a polymeric material. Fillers are added to modify properties such as mechanical and thermal properties. For example, carbon fibers can be added to reinforce polymers mechanically to enhance strength for certain uses, such as for load-bearing devices. In some embodiments, carbon-reinforced PEEK may be used. Carbon-filled PEEK is known to have enhanced compressive strength and stiffness, and a lower expansion rate relative to unfilled PEEK. Carbon-filled PEEK may also offer wear resistance and load-carrying capability.

In various embodiments, the present technology provides composites that comprise a ceramic, such as calcium-containing ceramics. Calcium-containing ceramics include those comprising, or consisting of, calcium carbonate, calcium sulfate, calcium lactobionate, calcium fluorite, calcium fluorophosphates, calcium chlorophosphate, calcium chloride, calcium lactate, hydroxyapatite, ceramics, calcium oxide, calcium monophosphate, calcium diphosphate, tricalcium phosphate, calcium silicate, calcium metasilicate, calcium silicide, calcium acetate, biphasic calcium phosphate, and mixtures thereof. Preferably the ceramic is absorbable, or is resorbable such that a substantial portion of the ceramic resorbs upon implantation in a human or animal subject, preferably within from about 6 to about 18 months after implantation. In various embodiments, the ceramic comprises, or is derived from, a natural source of calcium such as coral. In some embodiments, the ceramic comprises calcium carbonate, calcium phosphate, and combinations thereof.

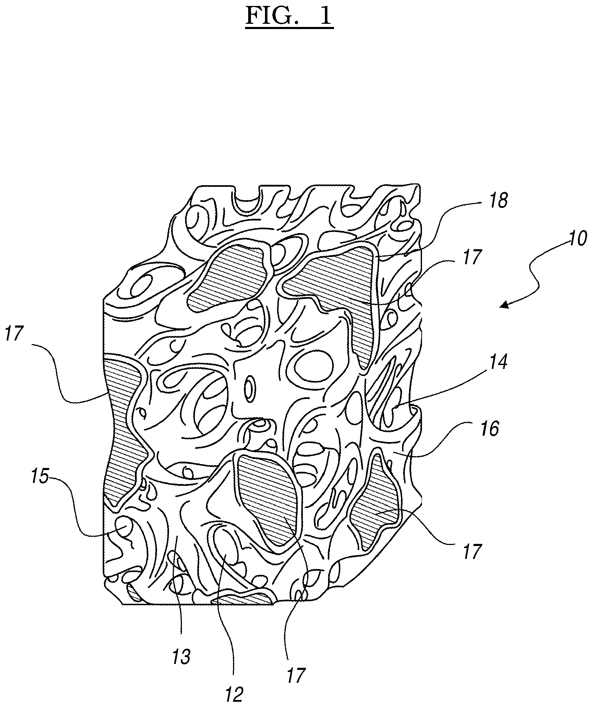

In various embodiments, the present technology provides composites and constructs comprising a porous structure comprising a non-resorbable polymer. In some embodiments, the constructs consist essentially of a non-resorbable polymer, i.e., containing no or low levels (e.g., less than 10%, less than 5%, or less than 1%) of ceramic or other structural materials. As exemplified in Figure 1, a porous structure 10 may comprise an interconnected strut structure, wherein the struts (e.g., struts 17) are substantially continuous throughout the construct. The porous structure 10 may be comprised of non-resorbable polymer or ceramic in various embodiments as discussed further herein. The strut structure defines a porosity comprising interconnected channels that are substantially continuous throughout the porous structure 10. One of ordinary skill in the art would understand, however, that such substantially continuous channels may not extend throughout the entirety of the construct, due to (for example) design of the construct or manufacturing variability. The interconnected channels generally extend through porous structure such that a path can be traced from a pore 12 on a first face 13 of the porous structure, into the porous structure, and exiting from one or more second pores 14, 15 on the first face 13 or another face 16 of the porous structure.

In various embodiments, the porous structure has a microstructure which approximates the same pore size as cancellous human bone, such that the porous structure is operable to allow permeation of body fluids and blood cells into the porous structure. Referring again to Figure 1, the porous structure 10 may include at least some macropores 12, 14, 15 communicating with the exterior surface (e.g., faces 13, 16) of the porous structure 10, of sufficient size to allow infiltration of blood vessels and other tissues and nutrients. The porous structure 10 may also include micropores, such as within the material of struts 17, which are pores too small in diameter to permit ingrowth of calcified bone tissue. The porous structure may comprise pores and channels having a size or transverse dimension (i.e., diameter or dimension transverse to the axis of the channel) of from about 5 to about 1000 microns, from 5 to about 800 microns, or from about 100 to about 700, or from about 400 to about 600 microns. In some embodiments, the dimension is about 500 microns.

The porous structure may be coralline, having a three-dimensional structure of struts substantially similar to the carbonate skeletal material of Scleractinia, or stony coral. Such coral include those of the genus Porites, Goniopora, Alveopora, and Acropora. The porous structure may also be "lost coralline" having a three-dimensional structure of struts substantially similar to the structure of internal channels in a coralline structure. Such a lost coralline structure may be characterized as the "negative" of a coralline structure, analogized to the structure produced by a "lost wax" - type casting using a coralline mold.

In some embodiments, at least a portion of the porosity of the structure, including interconnected channels of the structure, is wholly or partially filled with a ceramic. Such embodiments include composites comprising ceramic and non-resorbable polymer. Such composites can comprise:
a) a first phase comprising a ceramic; and
b) a second phase comprising a non-resorbable polymer;
c) wherein each of the first and second phases
   i) have an interconnected strut structure; and
   ii) are substantially continuous through the composite.
The first and second phases may have a porous structure as described above, such as a coralline or lost-coralline structure. In some embodiments, the first phase (ceramic) has a coralline structure and the second phase (polymer) has a lost-coralline structure. In other embodiments, the first phase has a lost-coralline structure and the second phase has a coralline structure. The first phase may comprise two or more ceramics in a multilayered structure of differing ceramic compositions. For example, the first phase may be a porous structure comprising interconnected struts comprising calcium carbonate coated with a layer of calcium phosphate. The layer of calcium phosphate may be from about 1 to about 15 microns, or from about 2 to about 10 microns, or from about 3 to about 8 microns in depth.

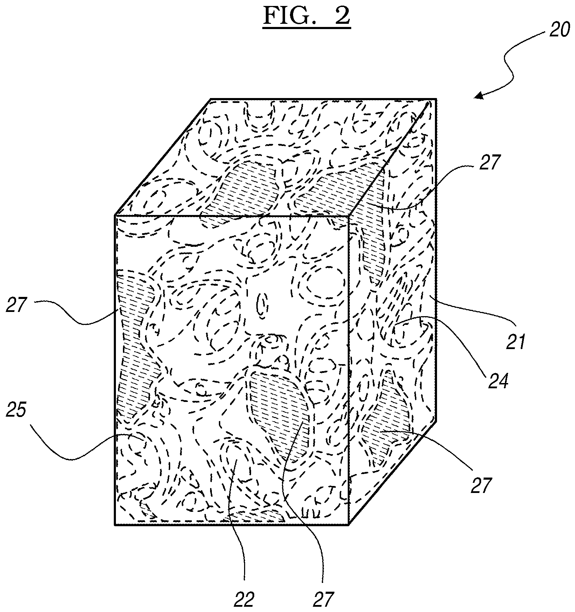

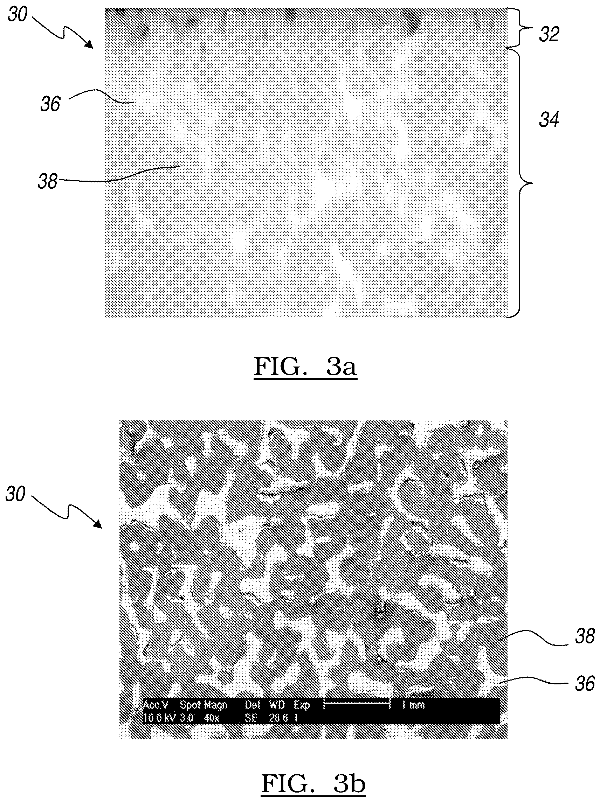

The structure of a ceramic/polymer composite is exemplified in Figure 2 and the photomicrographs of Figures 3a and 3b. The composite 20 of Figure 2 comprises a first phase, which is essentially the porous structure of Figure 1 comprising a ceramic, the porosity (e.g., pores 12, 14, 15) and interconnected channels of which have been substantially filled with a non-resorbable polymer 21 (e.g., PEEK) as a second phase, so as to form the composite 20 as a monolithic, substantially non-porous, two-phase composite block. In other embodiments, the first phase comprises a non-resorbable polymer and the second phase comprises a ceramic. In some embodiments, the first phase comprises a coralline structure, and the second phase comprises a lost-coralline structure. In other embodiments, the first phase comprises a lost-coralline structure, and the second phase comprises a coralline structure.

Figure 3a is a microphotograph of a cross-section of a composite 30 having a first phase 36 ceramic and second phase 38 non-resorbable polymer. As further exemplified, the composite 30 may optionally comprise a porous layer 32 of non-resorbable polymer extending from the second phase 38 non-resorbable polymer, on a surface of a core 34 that comprises the composite of the first phase 36 ceramic and the second phase 38 non-resorbable polymer. Preferably, as depicted in Figure 3a, the porous layer 32 and composite core 34 comprise the same non-resorbable polymer. In some embodiments, as depicted in Figure 3a, the porosity of the porous layer 32 is formed from channels in the non-resorbable polymer that are continuous with the interconnected channels of the second phase 38 non-resorbable polymer of the core 34. The depth of the porous layer 32 may be from about 0.05 to about 5 mm, from about 0.1 to about 3 mm, or from about 0.25 to about 1 mm.

Figure 3b is a scanning electron micrograph showing a section of the two-phases of a composite of the present technology. The composite 30 comprises a first phase 36 ceramic and a second phase 38 non-resorbable polymer, in a substantially solid, non-porous form.

The composites (as well as constructs) of the present technology can further comprise one or more bioactive materials. Depending on such factors as the bioactive material, the composition of the composite, the structure of the composite, and the intended use of the composite, the bioactive material may be coated on a surface of the composite, coated or otherwise infused in the pores (if any) of the composite, or mixed with the materials (e.g., non-porous polymer, ceramic, or both) of the composite. Bioactive materials can include any natural, recombinant or synthetic compound or composition that provides a local or systemic therapeutic benefit. In various embodiments, the bioactive material promotes the growth of bone directly or indirectly. Bioactive materials among those useful herein include isolated tissue materials, growth factors, peptides and other cytokines and hormones, pharmaceutical actives, and combinations thereof. Isolated tissue materials include, for example, whole blood and blood fractions (such as red blood cells, white blood cells, platelet-rich plasma, and platelet-poor plasma), bone marrow aspirate and bone marrow fractions, lipoaspirate and lipid-derived materials, isolated cells and cultured cells (such as hemopoietic stem cells, mesenchymal stem cells, endothelial progenitor cells, fibroblasts, reticulacytes, adipose cells, and endothelial cells). Growth factors and cytokines useful herein include transforming growth factor-beta (TGF-β) including the five different subtypes (TGF-β 1-5); bone morphogenetic factors (BMPs, such as BMP-2, BMP-2a, BMP-4, BMP-5, BMP-6, BMP-7 and BMP-8); platelet-derived growth factors (PDGFs); insulin-like growth factors (e.g., IGF I and II); and fibroblast growth factors (FGFs), vascular endothelial growth factor (VEGF), osteocalcin, osteopontin, and combinations thereof. Examples of pharmaceutical actives include antimicrobials, chemotherapeutic agents, and anti-inflammatories. Examples of antimicrobials include sulfonamides, furans, macrolides, quinolones, tetracyclines, vancomycin, cephalosporins, rifampins, aminoglycosides (such as tobramycin and gentamicin), and mixtures thereof.

### Implants and Methods of Treatment

The composites, constructs and implants of the present technology can be used in any of a variety of tissue defects. "Tissue defects" include any condition involving tissue which is inadequate for physiological or cosmetic purposes. Such defects include those that are congenital, those that result from or are symptomatic of disease (e.g., a degenerative disease) or trauma, and those that are consequent to surgical or other medical procedures. Such defects may be present in any aspect of the skeleton of a human or other animal subject, such as skull (including teeth and jaws), spine, and extremities (arms, legs, hands, and feet). Examples of tissue defects include skeletal or other bony tissue defects, such as those resulting from: osteoporosis; spinal fixation and fusion procedures; hip, knee, elbow and other joint replacement procedures; dental and craniomaxillofacial diseases, trauma and procedures; wounds; and fractures. Accordingly, the present technology provides methods for treating tissue defects in humans or other animals (not part of the invention) by implanting a composite or construct of the present technology at the site of the defect.

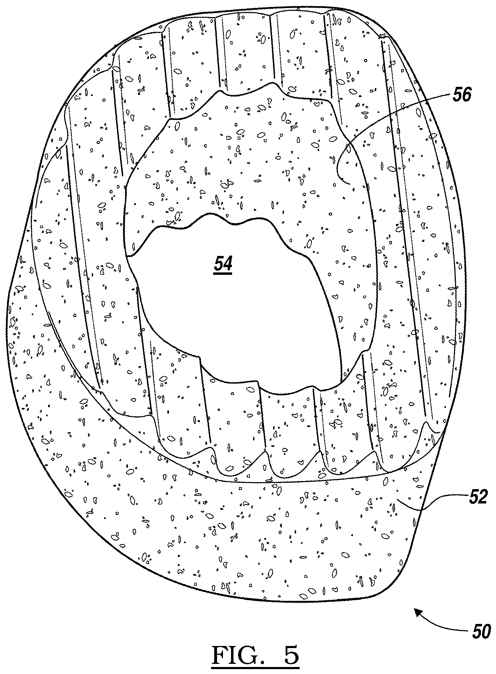

Implants of the present technology may consist essentially of a composite or construct of the present technology, or may comprise a composite or construct and other materials, components or devices depending on the intended use. In some embodiments, as further described below regarding methods of manufacturing, the present technology provides implants comprising two or more components, comprising a composite or construct of the present technology with another component which may comprise a material that is comprised in the composite or construct. Thus, for example, the present technology provides implants comprising a first component comprising a bone graft construct comprising (or consisting essentially of) a non-resorbable polymer, and a second non-porous component comprising the non-resorbable polymer, wherein the second component is contiguous with a surface of the first component. In some embodiments, the present technology provides implants comprising a composite comprising a ceramic and a non-resorbable polymer, and a non-porous component comprising (or consisting essentially of) the non-resorbable polymer, wherein the non- porous component is contiguous with a surface of the composite. Such implants may comprise a core comprising the composite, the core having an external surface; a porous layer, comprising the non-resorbable polymer, contiguous with the external surface of the core; and a non-porous component consisting essentially of the non-resorbable polymer; wherein the non-porous component is contiguous with a surface of the core, the porous layer, or both. Such implants are exemplified in Figures 5, 6, and 7, described below.

Without limiting the utility or function of the composites and constructs of the present technology, the ceramic component of composite is preferably gradually resorbable after implantation. For example, once implanted, the first phase (ceramic) of the composite may be gradually resorbed by osteoclasts allowing bone and blood vessels to penetrate into the center of the implant wall, and not just to particles exposed at the surface, as is the case with particulate composites. After implantation, the polymer component of the composite is not resorbable.

Preferably, once implanted, the non-resorbable polymer component affords load-bearing properties to the composite, providing support for other body structures, while allowing integration with the subject's native bone as the ceramic component is reabsorbed. As the ceramic portion degrades, stresses on the composite are transferred to the non-resorbable polymer, and the implant remains load-bearing. Such load-bearing composites have according to the invention compressive strength of from 50 to 150 MPa, or from about 90 to about 110 MPa. Composites containing higher preparation of non-resorbable polymer, particularly as non-porous posts or other solid regions, may have higher compressive strength, e.g., from 140-170 MPa. Implants having a non-porous polymer contiguous with a composite may also be useful where additional load-bearing strength is required, or in procedures for reconstruction of articulating joints where the solid polymer region is used as a bearing surface and the composite interfaces with bone.

Implants comprising composites and constructs of the present technology may be provided in any of a variety of forms, depending on their ultimate intended use. For example, the implants may have regular geometric shapes such as sheets, blocks, wedges, and cylinders, which may be machined or otherwise configured for use in a specific surgical procedure, either prior to or during the procedure. The implants may also be formed in shapes suitable for use in fixation procedures. Such shapes can include screws (such as interference screws), nails (such as tibial and other intramedullary nails, and arthrodesis nails), anchors, tacks, wires, and pins. The implants may also be formed in site-specific shapes useful in specific procedures. Such site- specific shapes include cervical spacers, lumbar spacers (e.g., for anterior lumbar interbody fusion or posterior lumbar interbody fusion procedures), spinal cages, bone plates, articulating surfaces (such as patellar implants), osteotomy wedges, spacers for replacing failed total ankle arthrodesis, cylinders for segmental defect repair, mandibular spacers, craniofacial spacers, and phalangeal spacers for digit lengthening.

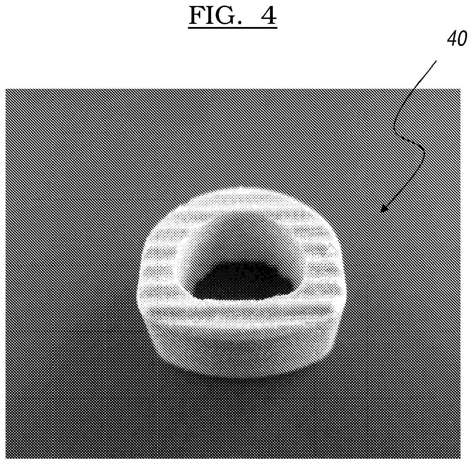

For example, referring to Figures 4, 5, 6, 7, and 8, spinal implants 40, 50, 60, 70, and 80 are depicted. Spinal implants 40, 50, 60, 70, and 80 may be for any appropriate spinal application, such as an intervertebral spacer for cervical fusion. The spinal implant 40, 50, 60, 70, and 80 may have a ring or open structure. For example, as depicted in Figure 5, the spinal implant 50 may include an exterior wall 52 and an interior void 54 defined by an interior wall 56. The interior void 54 can be operable to contain bone graft materials such as autograft, or allograft.

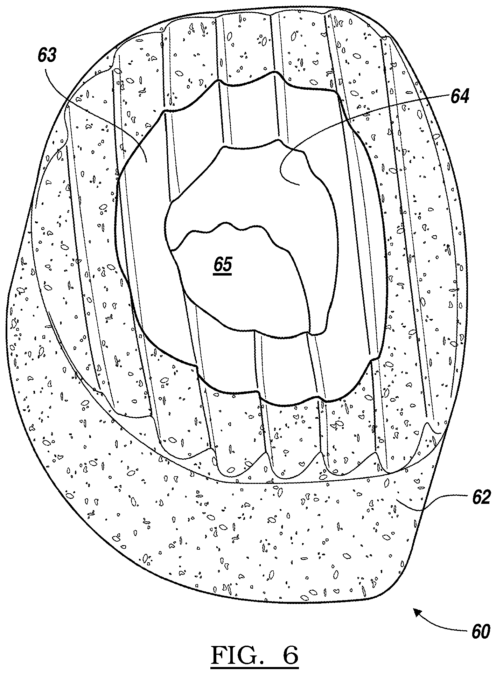

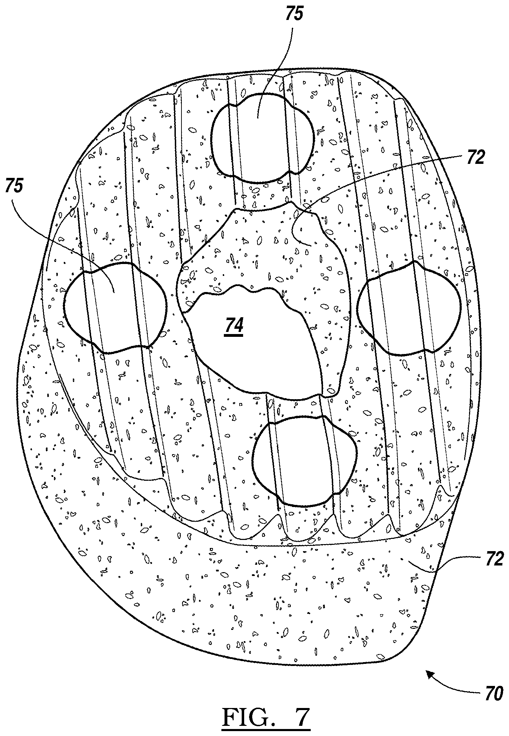

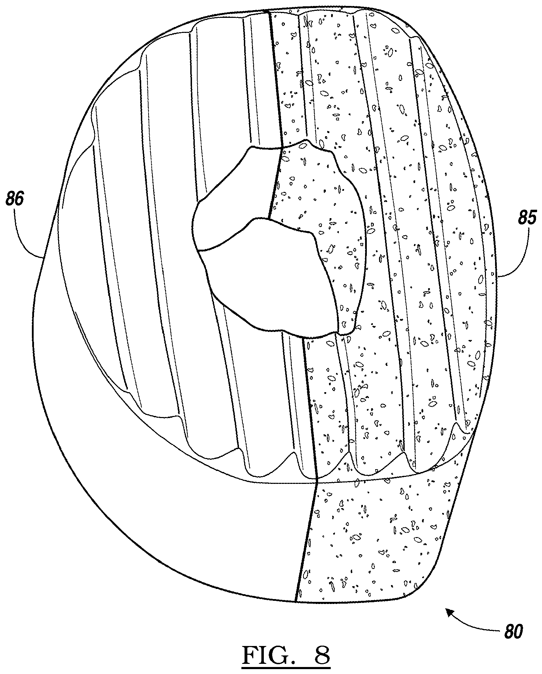

As discussed above, implants may comprise two or more components. As depicted in Figures 6, 7, and 8, such multi-component spinal implants 60, 70, 80 may comprise a polymer/ceramic composite 62, 72, 85 of the present invention, and a solid polymer component 63, 75, 86. For example, as depicted in Figure 6, the spinal implant 60 may comprise an inner annular ring 63 consisting essentially of solid polymer within an outer annular ring 62 comprising a composite. The inner annular ring 63 has an inner wall 64 that defines a void 65. As depicted in Figure 7, implant 70 may comprise one or more plugs 75 comprising solid non-resorbable polymer within a composite component 72. Further, as depicted in Figure 8, the spinal implant 80 may comprise a composite component 85 and a solid non-resorbable polymer component 86 which together form the implant 80. It should be noted, though, that the compositions of the solid component 63, 75, 86 and composite components 62, 72, 85 of the spinal implants 60, 70, 80 discussed above may be reversed in some embodiments such that, for example, the spinal implant 60 depicted in Figure 6 may comprise a polymer/ceramic composite component 63 and a solid polymer component 62.

### Methods of Manufacture

The composites and constructs of the present technology may be made by a variety of suitable methods, including methods comprising (a) infusing a non-resorbable polymer into a porous structure, or portion thereof, of a ceramic; or (b) infusing ceramic into a porous structure, or portion thereof, of non-resorbable polymer. By filling the porosity of the first phase with the second phase, the resulting composite consists essentially of two or more distinct, intact, and continuous phases forming a monolithic, substantially non-porous, structure.

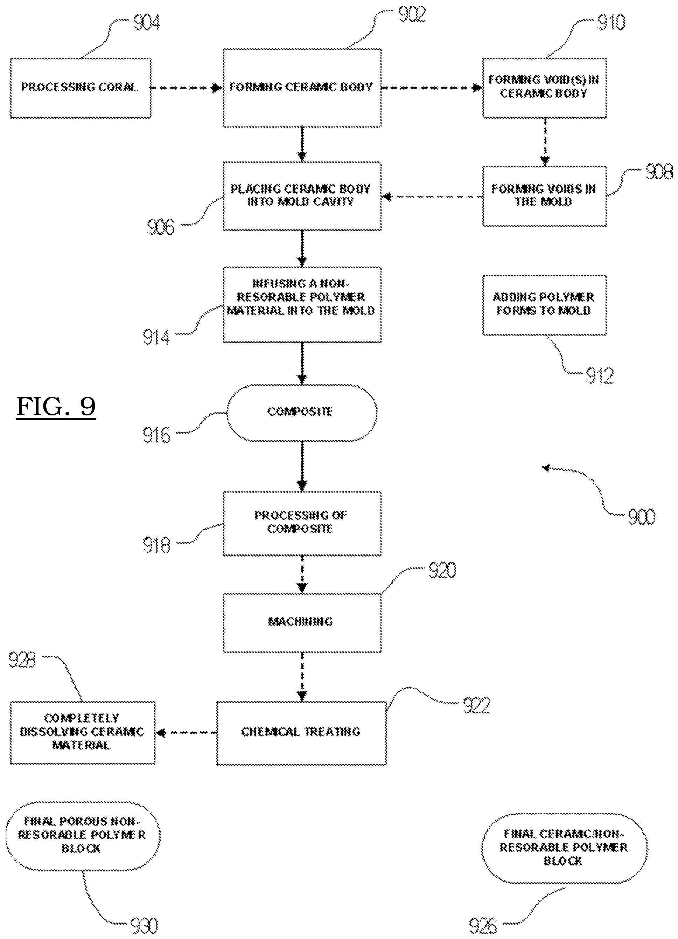

Referring to Figure 9, an exemplary method 900 comprises infusing a non-resorbable polymer into a porous ceramic body. In particular, the method comprises a ceramic forming step 902, comprising forming a ceramic having a plurality of interconnected channels. The method further comprises an infusing step 914, comprising substantially filling one or more of the interconnected channels of the ceramic body.

As discussed above, the ceramic body may have a coralline structure. Accordingly, in various embodiments, ceramic forming 902 comprises a coral processing step 904, comprising processing coral so as to make a ceramic body that is, or is derived from, coral skeletal material. As discussed above, such coral include those of the genus Porites, Goniopora, Alveopora, and Acropora.

Ceramic bodies derived from coral may consist essentially of the calcium carbonate and other minerals native to the coral, or may be processed so as to replace some or all of the native calcium with another calcium material. For example, the coral processing 904 may include chemically converting calcium carbonate in part, or in whole, to a calcium phosphate, such as hydroxyapatite. The conversion may be accomplished by a hydrothermal chemical exchange of carbonate with phosphate, by supplying an excess of phosphorus and oxygen to the coral material. The excess phosphorus can be supplied in the chemical form of phosphoric acid, ammonium phosphate, an organic phosphate, a phosphate salt such as a metal phosphate, or other, preferably water-soluble and volatilizable phosphate compounds. For example, coral processing 904 may comprise immersing a calcium carbonate coral in a bath of ammonium phosphate and heating (e.g., from about 200°C to about 250°C for a period of time), a hydrothermal chemical exchange reaction occurs in which the calcium carbonate body is converted to calcium phosphate.

Referring again to Figure 1, the conversion of calcium carbonate to calcium phosphate may be controlled so as to result in only partial conversion, forming a porous structure 10 ceramic comprising struts 17 of calcium carbonate coated with a layer 18 of calcium phosphate. The thickness of the layer 18 of calcium phosphate may be controlled by the reaction conditions. For example, if the reaction time is limited to from about 6 hours to about 12 hours, the porous structure 10 comprises struts 17 having a layer of calcium phosphate covering a calcium carbonate core. The resulting layer 18 of calcium phosphate on the interconnected struts of calcium carbonate may be from about 1 to about 15 microns, or from about 2 to about 10 microns, or from about 3 to about 8 microns in depth. Alternatively, the reaction time may be extended (e.g., from about 24 hours to about 60 hours to make a porous calcium body in which the calcium carbonate has been completely converted to calcium phosphate. Thus, in reference to Figure 1, there is not a layer 18 of calcium phosphate; rather, the entire structure consists essentially of uncoated struts 17 comprising calcium phosphate. It should be understood, however, that due to (for example) design or manufacturing variability, the coating of calcium phosphate may not be continuous throughout the internal structure of the calcium carbonate body, such that the resulting body may contain struts that are not coated with calcium phosphate.

Methods for converting calcium carbonate of coral to phosphate are described in U.S. Patent 3,929,971, Roy, issued December 30, 1975; U.S. Patent 4,976,736, White et al., issued December 11, 1990; and U.S. Patent 6,376,573, White et al, issued April 23, 2002. Such materials useful as ceramic bodies in the methods of this technology are commercially available, including Pro Osteon 500R and Pro Osteon HA (sold by Biomet, Inc., Warsaw, Indiana, USA, through one or more of its subsidiaries).

Referring back to Figure 9, as discussed above, the method includes infusing 914 non-resorbable polymer into the porosity of the ceramic body. "Infusing" includes any method by which a second phase material (e.g., non-resorbable polymer, as in the process of Figure 9) is introduced otherwise formed in pores and interconnected channels of a porous structure of a first phase material (e.g., ceramic, as in the process of Figure 9). It should be understood that there may be areas within the porous structure of the first phase material that are not infused with second phase material, polymer, either by design or due to (for example) manufacturing variability.

Infusing may comprise any of a variety of methods among those known in the art for introducing a material into pores, channels or other interstices of a second material. Infusing may comprise in-situ polymerization, wherein (for example) monomer or partially polymerized monomer is infused into the porosity of the ceramic, along with cross-linking agents, initiators or other materials as needed, followed by completion of the polymerization reaction to form the non-resorbable polymer.

In non-limiting reference to the process of Figure 9, infusing 914 may comprise injection molding of resorbable polymer into pores of the ceramic body. As exemplified in Figure 9, the method may comprise a placing step 906, comprising placing the ceramic body in a mold. The infusing step 914 then comprises injecting molten polymer into the mold under sufficient force so as to penetrate the porosity of the ceramic body.

In other methods, infusing 914 may comprise compression molding. Vacuum impregnation techniques may also be used, whereby a relatively low pressure is formed in the ceramic body so as to pull the polymer into the porosity. In some embodiments, the porous ceramic body is immersed in a liquid medium of the non-resorbable polymer, followed by hardening or in-situ polymerization. Other techniques for infusing 914 include solution embedding, where the polymer is dissolved in a suitable solvent, and then cast into the mold so as to fill porosity of the ceramic body.

In various embodiments, the placing step 906 comprises a void forming step 910, wherein a void is defined by a surface of the ceramic body and the interior surface of the mold. Thus, in such methods, the mold has a volume greater than the volume of the ceramic body, such that the body defines a void external to the ceramic body in the mold. In such methods, the infusing 914 comprises injecting or otherwise infusing the non-porous polymer into the mold so as to substantially fill the void and one or more channels of the ceramic body. The void may be external to the ceramic body (i.e., outside the surface faces of the body) or, in some methods, the void forming 910 comprises forming voids internal to the ceramic body. Such internal voids are distinct from the pores and interconnected channels of the ceramic body, and include such features as cavities and channels. In some embodiments, the placing step 906 further comprises placing one or more solid blocks or other forms of solid non-resorbable polymer are placed in the void prior to infusing 914 the non-porous polymer into the mold.

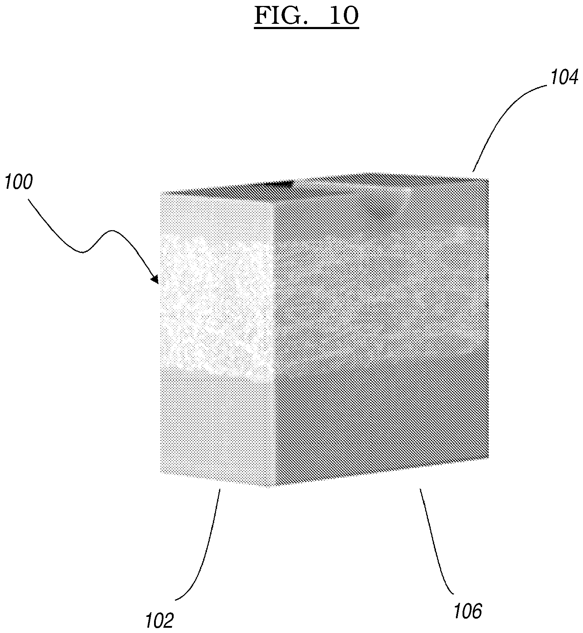

As exemplified in Figure 10, an implant 100 made by such a process can comprise a composite 102 having a first phase comprising a ceramic (i.e., the ceramic of the ceramic body), and a second phase comprising a non-resorbable polymer (i.e., infused into the ceramic body), wherein each of the first and second phases have an interconnected strut structure and are substantially continuous through the composite. As discussed above, an implant may comprise the ceramic/non-resorbable polymer composite with an additional component comprising (or consisting essentially of) the non-porous polymer. Such an implant 100 further comprises a non-porous component 104 (i.e., formed in the void) comprising the non-resorbable polymer, wherein the non-porous component is contiguous with a surface of the composite. With further reference to Figure 9, as well as Figure 10, implants 100 made by a method 900 in which a form of solid polymer is placed in a mold during the placing step 906, as discussed above, comprise a non-porous component 104 comprising the non-porous polymer infused during the infusing step 914 as well as the solid polymer form 106.

In some embodiments, the ceramic body comprises a first face and a second face opposing the first face, and the void forming step 910 comprises forming a passage in the ceramic body connecting the first face to the second face. Preferably, the passage void has a transverse dimension (e.g., diameter) that is at least ten times greater than the transverse dimension of the interconnected channels of the ceramic body. Implants comprising composites 916 made by such methods include those comprising a post of the non-resorbable polymer extending from the first face to the second face and formed in the passage during the injecting. Such embodiments are exemplified in Figure 7, discussed above.

The methods may further comprise a processing step 918 after infusing 914 of the non-resorbable polymer. The processing 918 may comprise machining 920 the composite 916 into a final form, suitable for implantation into human or other animal subject, or combining with other materials or devices to construct an implant.

Processing 918 may also comprise chemically treating 922 the composite 916 to alter its chemical or physical structure. For example, methods may further comprise selectively dissolving ceramic from the composite 916, using one or more solvents in which the first phase (ceramic) of the composite 916 is soluble, but the second phase (non-resorbable polymer) of the composite 916 is not soluble. Such solvents include organic acids such as formic acid, oxalic acid, and acetic acid, and inorganic acids such as hydrochloric acid, nitric acid, sulfuric acid, and phosphoric acid. By controlling the pH of the acid bath, as well as the time of exposure, the first phase may be dissolved entirely or partially to a desired depth.

In further reference to Figure 9, as well as Figure 3a, in some methods 900, chemical treating 922 involves partially dissolving 924 the ceramic so as to form a final ceramic/polymer composite 926 having a surface layer 32 of porous non-resorbable polymer on a core 34 of ceramic and polymer. The interconnected strut structure of the first phase ceramic in the surface of the composite 916, 30 is removed during the partial dissolving 924, leaving the interconnected strut structure of the second phase non-resorbable polymer in the surface layer 32. The voids created by removal of the interconnected struts of ceramic thus form interconnected channels in the non-porous polymer of the surface layer 32. For example, an exposure time of about 10 to about 60 minutes to the solvent will partially dissolve the first phase ceramic at the surface of the composite 916. The depth of the resulting porous layer 32 may be from about 0.05 to about 5 mm, from about 0.1 to about 3 mm, or from about .25 to about 1 mm.

Composites made using a bi-phasic porous ceramic body, comprising struts of calcium carbonate coated with a layer calcium phosphate, may be treated to selectively dissolve the calcium carbonate from the first phase ceramic at the surface, while leaving some or all of the calcium phosphate. It will be appreciated by one of ordinary skill in the art that selection of acid, such as acetic acid, and control of reaction time and conditions will allow preferential dissolution of calcium carbonate. The resulting composite 926 comprises a porous outer layer of non-resorbable polymer with interconnected struts coated with calcium phosphate, the calcium carbonate of the outer layer having been removed to form interconnected channels. In such embodiments, the remaining layer of calcium phosphate may be from about 1 to about 15 microns, or from about 2 to about 10 microns, or from about 3 to about 8 microns in depth.

In some embodiments, the chemical treating may involve completely dissolving 924 the ceramic so as to remove all, or essentially all, of the first phase ceramic. A stronger acid, such as hydrochloric acid, may be used to accelerate removal of ceramic. The resulting porous body 930 consists or consists essentially of non-resorbable polymer. Such a construct 930 may comprise a non-resorbable polymer having a lost-coralline structure, wherein the ceramic body used in making the construct had a coralline structure. (It should be understood that other methods may be used to make lost-coralline constructs comprising non-porous polymer.)

### Non-limiting Discussion of Terminology

The headings (such as "Introduction" and "Summary") and subheadings used herein are intended only for general organization of topics within the present disclosure, and are not intended to limit the disclosure of the technology or any aspect thereof. In particular, subject matter disclosed in the "Introduction" may include novel technology and may not constitute a recitation of prior art. Subject matter disclosed in the "Summary" is not an exhaustive or complete disclosure of the entire scope of the technology or any embodiments thereof. Classification or discussion of a material within a section of this specification as having a particular utility is made for convenience, and no inference should be drawn that the material must necessarily or solely function in accordance with its classification herein when it is used in any given composition.

The description and specific examples, while indicating embodiments of the technology, are intended for purposes of illustration only and are not intended to limit the scope of the technology. Moreover, recitation of multiple embodiments having stated features is not intended to exclude other embodiments having additional features, or other embodiments incorporating different combinations of the stated features. Specific examples are provided for illustrative purposes of how to make and use the compositions and methods of this technology and, unless explicitly stated otherwise, are not intended to be a representation that given embodiments of this technology have, or have not, been made or tested.

As used herein, the words "prefer" or "preferable" refer to embodiments of the technology that afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the technology.

As used herein, the word "include," and its variants, is intended to be non-limiting, such that recitation of items in a list is not to the exclusion of other like items that may also be useful in the materials, compositions, devices, and methods of this technology. Similarly, the terms "can" and "may" and their variants are intended to be non-limiting, such that recitation that an embodiment can or may comprise certain elements or features does not exclude other embodiments of the present technology that do not contain those elements or features.

Although the open-ended term "comprising," as a synonym of non- restrictive terms such as including, containing, or having, is used herein to describe and claim embodiments of the present technology, embodiments may alternatively be described using more limiting terms such as "consisting of or "consisting essentially of." Thus, for any given embodiment reciting materials, components or process steps, the present technology also specifically includes embodiments consisting of, or consisting essentially of, such materials, components or processes excluding additional materials, components or processes (for consisting of) and excluding additional materials, components or processes affecting the significant properties of the embodiment (for consisting essentially of), even though such additional materials, components or processes are not explicitly recited in this application. For example, recitation of a composition or process reciting elements A, B and C specifically envisions embodiments consisting of, and consisting essentially of, A, B and C, excluding an element D that may be recited in the art, even though element D is not explicitly described as being excluded herein. Further, as used herein the term "consisting essentially of' recited materials or components envisions embodiments "consisting of' the recited materials or components.

As referred to herein, ranges are, unless specified otherwise, inclusive of endpoints and include disclosure of all distinct values and further divided ranges within the entire range. Thus, for example, a range of "from A to B" or "from about A to about B" is inclusive of A and of B. Disclosure of values and ranges of values for specific parameters (such as temperatures, molecular weights, weight percentages, etc.) are not exclusive of other values and ranges of values useful herein. It is envisioned that two or more specific exemplified values for a given parameter may define endpoints for a range of values that may be claimed for the parameter. For example, if Parameter X is exemplified herein to have value A and also exemplified to have value Z, it is envisioned that Parameter X may have a range of values from about A to about Z. Similarly, it is envisioned that disclosure of two or more ranges of values for a parameter (whether such ranges are nested, overlapping or distinct) subsume all possible combination of ranges for the value that might be claimed using endpoints of the disclosed ranges. For example, if Parameter X is exemplified herein to have values in the range of 1-10, or 2-9, or 3-8, it is also envisioned that Parameter X may have other ranges of values including 1-9, 1-8, 1-3, 1-2, 2-10, 2-8, 2-3, 3-10, and 3-9.

## Claims

1. An orthopedic composite, the composite comprising:
a) a first phase comprising a ceramic that is coral or that is derived from coral via hydrothermal conversion of calcium carbonate in the coral to a calcium phosphate, wherein the first phase has a porous coralline structure; and
b) a second phase comprising a non-resorbable polymer;
c) wherein each of the first and second phases
i) have an interconnected strut structure; and
ii) are continuous through the composite wherein the orthopedic composite has a compressive strength of 50 MPa to 150 MPa.

2. The orthopedic composite according to Claim 1, wherein the first phase comprises calcium carbonate coated with calcium phosphate.

3. The orthopedic composite according to Claim 1, wherein the non-resorbable polymer is infused into the coralline structure of the first phase so that the second phase has a lost-coralline structure.

4. The orthopedic composite according to Claim 1, wherein the non-resorbable polymer comprises PEEK.

5. The orthopedic composite according to Claim 1, comprising:
a) a core comprising the orthopedic composite; and
b) a porous layer, comprising the non-resorbable polymer, on a surface of the core, wherein the porous layer is from 0.1 mm to 1 mm in depth.

6. The orthopedic composite according to Claim 1, further comprising a non-porous component comprising the non-resorbable polymer, wherein the non-porous component is contiguous with a surface of the composite, further wherein the composite has a first face and a second face opposing the first face, and the surface of the composite defines a post of the non-resorbable polymer connecting the first and second faces.

7. The orthopedic composite according to Claim 1, comprising
a) a core comprising the composite, the core having an external surface;
b) a porous layer, comprising the non-resorbable polymer, contiguous with the external surface of the core; and
c) a non-porous component consisting of the non- resorbable polymer; wherein the non-porous component is contiguous with a surface of the core, the porous layer, or both.

8. A method of making an orthopedic composite according to claim 1, comprising infusing a porous ceramic body that is coral or that is derived from coral via hydrothermal conversion of calcium carbonate in the coral to a calcium phosphate, having a coralline structure and a plurality of interconnected channels, with a non-resorbable polymer, wherein the non-resorbable polymer optionally comprises PEEK, wherein the orthopedic composite has a compressive strength of 50 MPa to 150 MPa.

9. The method of making an orthopedic composite according to Claim 8, wherein the composite comprises
a) a first phase comprising the ceramic;
b) a second phase comprising the non-resorbable polymer; and
c) the first and second phases are continuous through the composite.

10. The method of making an orthopedic composite according to Claim 8, further comprising dissolving at least a portion of the ceramic material.

11. The method of making an orthopedic composite according to Claim 10, wherein the ceramic material is completely dissolved, such that the composite consists of porous polymer.

12. The method of making an orthopedic composite according to Claim 10, wherein the dissolving removes the ceramic material at the surface of the composite to a depth of from 0.1 to 1 mm.

13. The method of making an orthopedic composite according to Claim 8, wherein the porous ceramic body has a coralline structure, such that the bone graft composite comprises lost-coralline porous polymer structure, further wherein the porous ceramic body comprises calcium carbonate and one or more of the channels are coated with calcium phosphate.

14. The method of making an orthopedic composite according to Claim 13, further comprising dissolving the calcium carbonate at the surface of the composite, resulting in a porous polymer structure at the comprising interconnected channels having a coating of calcium phosphate.

15. The method of making an orthopedic composite according to Claim 8, wherein the infusing comprises injecting the polymer into the porous ceramic body filling one or more of the interconnected channels, wherein
(a) the implant comprises the composite and a second non-porous component comprising the non-resorbable polymer;
(b) the second component is contiguous with a surface of the first component the injecting comprises placing the ceramic body into a mold;
(c) the porous ceramic body defines a void external to the body in the mold; and
(d) infusing comprises injecting the non-resorbable polymer into the mold so as to fill the void and one or more channels of the porous ceramic body.

## Patentansprüche

1. Orthopädisches Verbundmaterial, wobei das Verbundmaterial umfasst:
a) eine erste Phase, die eine Keramik umfasst, die eine Koralle ist oder die von einer Koralle über hydrothermische Umwandlung von Kalziumcarbonat in der Koralle zu einem Calciumphosphat abgeleitet ist, wobei die erste Phase eine poröse korallenartige Struktur aufweist;
b) eine zweite Phase, die ein nicht resorbierbares Polymer umfasst;
c) wobei jede von der ersten und zweiten Phase
i) eine miteinander verbundene Strebenstruktur aufweist; und
ii) durch das Verbundmaterial hindurch kontinuierlich sind, wobei das orthopädische Verbundmaterial eine Druckfestigkeit von 50 MPa bis 150 MPa aufweist.

2. Orthopädisches Verbundmaterial nach Anspruch 1, wobei die erste Phase Calciumcarbonat umfasst, das mit Calciumphosphat beschichtet ist.

3. Orthopädisches Verbundmaterial nach Anspruch 1, wobei das nicht resorbierbare Polymer in die Korallenstruktur der ersten Phase eingedrungen ist, so dass die zweite Phase eine Struktur mit verlorener korallenartiger Struktur aufweist.

4. Orthopädisches Verbundmaterial nach Anspruch 1, wobei das nicht resorbierbare Polymer PEEK umfasst.

5. Orthopädisches Verbundmaterial nach Anspruch 1, umfassend:
a) einen Kern, der das orthopädische Verbundmaterial umfasst; und
b) eine poröse Schicht, die das nicht resorbierbare Polymer umfasst, an einer Fläche des Kerns, wobei die poröse Schicht eine Tiefe von 0,1 mm bis 1 mm aufweist.

6. Orthopädisches Verbundmaterial nach Anspruch 1, ferner mit einer nicht porösen Komponente, die das nicht resorbierbare Polymer umfasst, wobei die nicht poröse Komponente an eine Fläche des Verbundmaterials angrenzt, wobei ferner das Verbundmaterial eine erste Seite und eine zweite Seite, die der ersten Seite gegenüberliegt, aufweist, und die Fläche des Verbundmateriales einen Pfosten des nicht resorbierbaren Polymers definiert, der die erste und zweite Seite verbindet.

7. Orthopädisches Verbundmaterial nach Anspruch 1, umfassend:
a) einen Kern, der das Verbundmaterial umfasst, wobei der Kern eine Außenfläche aufweist;
b) eine poröse Schicht, die das nicht resorbierbare Polymer umfasst und an die Außenfläche des Kerns angrenzt; und
c) eine nicht poröse Komponente, die aus dem nicht resorbierbaren Polymer besteht;
wobei die nicht poröse Komponente an eine Fläche des Kerns, die poröse Schicht oder beide angrenzt.

8. Verfahren zum Herstellen eines orthopädischen Verbundmateriales nach Anspruch 1, mit einem Durchdringen eines porösen Keramikkörpers, der eine Koralle ist oder von einer Koralle über hydrothermische Umwandlung von Calciumcarbonat in der Koralle zu einem Calciumphosphat abgeleitet ist, der eine korallenartige Struktur und eine Mehrzahl verbundener Kanäle aufweist, mit einem nicht resorbierbaren Polymer, wobei das nicht resorbierbare Polymer optional PEEK umfasst, wobei das orthopädische Verbundmaterial eine Druckfestigkeit von 50 MPa bis 150 MPa aufweist.

9. Verfahren zum Herstellen eines orthopädischen Verbundmaterials nach Anspruch 8, wobei das Verbundmaterial umfasst
a) eine erste Phase, die die Keramik umfasst;
b) eine zweite Phase, die das nicht resorbierbare Polymer umfasst; und
c) wobei die erste und zweite Phase durch das Verbundmaterial kontinuierlich sind.

10. Verfahren zum Herstellen eines orthopädischen Verbundmateriales nach Anspruch 8, ferner mit einem Lösen zumindest eines Teils des Keramikmaterials.

11. Verfahren zum Herstellen eines orthopädischen Verbundmaterials nach Anspruch 10, wobei das Keramikmaterial vollständig gelöst ist, so dass das Verbundmaterial aus einem porösen Polymer besteht.

12. Verfahren zum Herstellen eines orthopädischen Verbundmaterials nach Anspruch 10, wobei das Lösen das Keramikmaterial an der Fläche des Verbundmateriales bis zu einer Tiefe von 0,1 bis 1 mm entfernt.

13. Verfahren zum Herstellen eines orthopädischen Verbundmaterials nach Anspruch 8, wobei der poröse Keramikkörper eine korallenartige Struktur aufweist, so dass ein Knochenpfropfen-Verbundmaterial eine poröse Polymerstruktur mit verlorener korallenartiger Struktur umfasst, wobei ferner der poröse Keramikkörper Calciumcarbonat umfasst und ein oder mehrere der Kanäle mit Calciumphosphat beschichtet sind.

14. Verfahren zum Herstellen eines orthopädischen Verbundmaterials nach Anspruch 13, ferner mit einem Lösen des Calciumcarbonats an der Fläche des Verbundmateriales, was in einer porösen Polymerstruktur an den einschließenden verbundenen Kanälen, die eine Beschichtung aus Calciumphosphat aufweisen, resultiert.

15. Verfahren zum Herstellen eines orthopädischen Verbundmaterials nach Anspruch 8, wobei das Durchdringen ein Injizieren des Polymers in dem porösen Keramikkörper umfasst, wobei ein oder mehrere der verbundenen Kanäle gefüllt werden, wobei
(a) das Implantat das Verbundmaterial und eine zweite nicht poröse Komponente umfasst, die das nicht resorbierbare Polymer umfasst;
(b) die zweite Komponente an eine Fläche der ersten Komponente angrenzt, wobei das Injizieren umfasst, dass der Keramikkörper in einer Form platziert ist;
(c) der poröse Keramikkörper einen Hohlraum außerhalb des Körpers in der Form definiert; und
(d) das Durchdringen ein Injizieren des nicht resorbierbaren Polymers in die Form umfasst, um den Hohlraum und einen oder mehrere Kanäle des porösen Keramikkörpers zu füllen.

## Revendications

1. Composite orthopédique, le composite comprenant :
a) une première phase comprenant une céramique qui est du corail ou qui est dérivée à partir de corail via une conversion hydrothermique du carbonate de calcium dans le corail pour former un phosphate de calcium, dans lequel la première phase a une structure corallienne poreuse ; et
b) une seconde phase comprenant un polymère non résorbable ;
c) dans lequel chacune de la première et de la seconde phase
i) a une structure à entretoises interconnectées ; et
ii) est continue à travers le composite, et le composite orthopédique présente une résistance à la compression de 50 MPa à 150 MPa.

2. Composite orthopédique selon la revendication 1, dans lequel la première phase comprend du carbonate de calcium revêtu de phosphate de calcium.

3. Composite orthopédique selon la revendication 1, dans lequel le polymère non résorbable est infusé dans la structure corallienne de la première phase de telle façon que la seconde phase a une structure corallienne perdue.

4. Composite orthopédique selon la revendication 1, dans lequel le polymère non résorbable comprend du PEEK.

5. Composite orthopédique selon la revendication 1, comprenant :
a) un noyau comprenant le composite orthopédique ; et
b) une couche poreuse, comprenant le polymère non résorbable, sur une surface du noyau, dans lequel la couche poreuse a une profondeur de 0,1 mm à 1 mm.

6. Composite orthopédique selon la revendication 1, comprenant en outre un composant non poreux comprenant le polymère non résorbable, dans lequel le composant non poreux est contigu à une surface du composite, et en outre dans lequel le composite a une première face et une seconde face à l'opposé de la première face, et la surface du composite définit un pilier du polymère non résorbable qui connecte la première et la seconde face.

7. Composite orthopédique selon la revendication 1, comprenant
a) un noyau comprenant le composite, le noyau ayant une surface externe ;
b) une couche poreuse, comprenant le polymère non résorbable, contiguë à la surface extérieure du noyau ; et
c) un composant non poreux constitué du polymère non résorbable ; dans lequel le composant non poreux est contigu à une surface du noyau, de la couche poreuse, ou des deux.

8. Procédé pour produire un composite orthopédique selon la revendication 1,
comprenant l'opération consistant à infuser un corps en céramique poreuse qui est du corail ou qui est dérivé à partir de corail via une conversion hydrothermique du carbonate de calcium dans le corail pour donner un phosphate de calcium, ayant une structure corallienne et une pluralité de canaux interconnectés, avec un polymère non résorbable, dans lequel le polymère non résorbable comprend en option du PEEK, dans lequel le composite orthopédique présente une résistance à la compression de 50 MPa à 150 MPa.

9. Procédé pour produire un composite orthopédique selon la revendication 8, dans lequel le composite comprend
a) une première phase comprenant la céramique ;
b) une seconde phase comprenant le polymère non résorbable ; et
c) la première et la seconde phase sont continues à travers le composite.

10. Procédé pour produire un composite orthopédique selon la revendication 8, comprenant en outre l'opération consistant à dissoudre au moins une portion du matériau céramique.

11. Procédé pour produire un composite orthopédique selon la revendication 10, dans lequel le matériau céramique est complètement dissous, de telle façon que le composite est constitué de polymère poreux.

12. Procédé pour produire un composite orthopédique selon la revendication 10, dans lequel l'étape de dissolution supprime le matériau céramique à la surface du composite sur une profondeur de 0,1 à 1 mm.

13. Procédé pour produire un composite orthopédique selon la revendication 8, dans lequel le corps en céramique poreuse a une structure corallienne, de telle façon que le composite pour greffe osseuse comprend une structure polymère poreuse corallienne perdue, et en outre dans lequel le corps en céramique poreuse comprend du carbonate de calcium, et un ou plusieurs des canaux sont revêtus avec du phosphate de calcium.

14. Procédé pour produire un composite orthopédique selon la revendication 13, comprenant en outre l'opération consistant à dissoudre le carbonate de calcium à la surface du composite, avec pour résultat une structure polymère poreuse comprenant des canaux interconnectés ayant un revêtement de phosphate de calcium.

15. Procédé pour produire un composite orthopédique selon la revendication 8, dans lequel l'opération d'infusion comprend l'injection du polymère dans le corps en céramique poreuse en remplissant un ou plusieurs des canaux interconnectés, dans lequel
a) l'implant comprend le composite et un second composant non poreux comprenant le polymère non résorbable ;
b) le second composant est contigu à une surface du premier composant, l'injection comprenant l'opération consistant à placer le corps céramique dans un moule ;
c) le corps en céramique poreuse défini un vide à l'extérieur du corps dans le moule ; et
d) l'opération d'infusion comprend l'injection du polymère non résorbable dans le moule de manière à remplir le vide et un ou plusieurs canaux du corps en céramique poreuse.
